# EUROPEAN PATENT SPECIFICATION

(11) **EP 2 219 556 B1**
(45) Date of publication and mention of the grant of the patent: **08.07.2015**
(21) Application number: 08849007.3
(22) Date of filing: 17.11.2008
(51) Int. Cl.: A61F 2/06, A61M 25/01, A61M 29/00

(54) **HYBRID INTRALUMINAL DEVICE**
INTRALUMINALE HYBRIDVORRICHTUNG
DISPOSITIF INTRALUMINAL HYBRIDE

(30) Priority: 15.11.2007 AU 2007906268
(43) Date of publication of application: 25.08.2010
(73) Proprietor: Gore Enterprise Holdings, Inc., Newark, DE 19714 (US)
(72) Inventor: WHITE, Geoffrey, H., Birchgrove NSW 2041 (AU); YU, Weiyan, Birchgrove NSW 2041 (AU)
(74) Representative: Shanks, Andrew
(86) International application number: PCT/AU2008/001710
(87) International publication number: WO 2009/062264

(56) References cited:
- EP-A2- 0 965 311
- WO-A1-00/76423
- WO-A1-01/24735
- WO-A1-99/60953
- WO-A1-03/020175
- WO-A1-03/082152
- WO-A1-2007/059280
- WO-A1-2007/124053
- WO-A2-01/74270
- WO-A2-02/36045
- WO-A2-99/60915
- WO-A2-03/063729
- WO-A2-2007/079067
- US-A- 6 123 723
- US-A1- 2003 105 516
- US-A1- 2004 098 087
- US-A1- 2007 010 874

## Description

### Field of the Invention

The present invention relates to an intraluminal device and particularly to a device for placement in an artery.

### Background Art

An artery or other vessel that is weakened by disease, injury, or congenital defect, can become distended due to the pressure of blood or other fluid flowing through the weakened area. In the vasculature, this distended weakening is called an aneurysm. An aneurysm typically occurs in the arterial vessels of the head, chest, or abdomen. The distension may cause the vessel to rupture.

Aneurysms in the abdominal or thoracic aorta are typically distended around the circumference of the aorta and tapered at both ends. Most aneurysms are caused by atherosclerotic or degenerative weakening of a segment of the wall, sometimes associated with congenital disorders or with trauma to the vessel. Abdominal aneurysms may cause backache and severe pain, and may be visible as a throbbing swelling. Rupture of an abdominal, thoracic, iliac or cerebral aneurysm is life threatening.

Traditionally, aneurysms have been treated by radical surgical graft replacement. This approach is risky for the patient and is sometimes not feasible due to other pre-existing disease states of the patient. More recently, aneurysms have been treated by placement of an intraluminal or endovascular graft. These intraluminal or endovascular grafts may be of various types, including grafts having stents, wireforms, or other attachment means attached to or integrated into the graft structure.

In general, intraluminal grafts and their respective support and/or attachment means fall into two major categories, self-expanding and pressure expandable. Self-expanding intraluminal grafts, are supported and/or attached via resilient or shape-memory material such as spring steel or Nitinol^{™}. Self-expanding material is capable of being formed in a configuration from which it may be compressed to a radially compact diameter for placement within a damaged vessel. At the time of use, the inherent or memory features of these materials causes them to self-expand from the radially compact diameter to the expanded nominal operative diameter. Many can then be further expanded by balloon pressure, to a variable extent, but some will often tend to recoil back towards a nominal diameter, depending on features such as material, wire dimensions and pattern. Wireforms or stents used for the purpose of expanding these grafts typically have variable elements of both self-expansion and balloon-expandability, so that a "self-expanding" wireform will have a major element of shape memory recoil, and a minor aspect of further response to pressure expansion, whereas a "balloon-expandable" wireform will typically have minor recoil or spring function, and a major component of hoop strength after mechanical expansion.

Typically, a graft with a nominal diameter greater than that of the target vessel is used so that it exerts an outward expansile force, resulting in good vessel wall apposition. The ultimate diameter of the vessel with the graft is the result of the equilibrium between the elastic recoil of the vessel and the radial expansion force of the graft. Self expanding grafts, have a tendency to foreshorten and recoil within the vessel. Endovascular grafts supported by a series of self-expanding wireforms or stents will typically be limited to a resultant straight cylindrical shape, with rounded or circular cross-sectional outline, so that they will not necessarily conform well to the inner wall of vessels which are irregular in shape, angulated or tapered. This is particularly a problem in regions of irregular diameter coinciding with angulation, curvature or tortuosity of the vessel, such as is frequently seen in the thoracic aortic arch, the proximal part of descending thoracic aorta, the suprarenal and Infrarenal segments of aortic wall associated with abdominal aortic aneurysms, and the iliac arteries To counteract the recoil of a self-expanding graft in anatomical regions such as these, surgeons may follow up with the introduction of a bare, balloon expandable stent implanted within that segment of graft, to force the graft radially outwardly and into an improved vessel wall apposition. The introduction of a rigid, bare stent complicates the procedure and may cause damage to the fabric structure of the implanted graft. Pressure-expandable intraluminal grafts are supported and/or attached via plastically deformable material such as stainless steel or Elgiloy that is initially formed in its radially compact diameter. This type of material has only a minor element of memory, and will remain close to its radially compact diameter until manually expanded. Typically, outwardly directed pressure is exerted upon the graft through use of a balloon so as to cause radial expansion and resultant plastic deformation of the material to its operative diameter. The plastic nature of these grafts provides a resistance against elastic recoil of the vessel and balloon-expandable grafts typically have greater radial and hoop strength than self-expanding grafts. This results in a greater conformability of the outer wall of the graft to any contour or shape changes of the inner wall of a vessel. The plastic nature of these grafts, however, renders them susceptible to irreversible deformation when subjected to a powerful external compressive force.

Careful positioning and firm implantation, attachment and seal of the intraluminal graft in the regions of adherence to the vessel wall are critical to the successful treatment of the underlying medical condition. This is particularly difficult to accomplish when the aneurysm extends from an artery into one or more divergent arteries or where the aneurysm causes an irregularly shaped neck of aorta. The conventionally used grafts, both self-expanding and pressure-expandable, have recognized shortcomings that make them less than complete solutions to the treatment of aneurysms in the vasculature, or to the treatment of similar damage to other vessels. The present invention provides substantial improvements to the methods and apparatus of the prior art.

A particular limitation of current self-expanding endoluminal grafts is that the circular pattern of self-expanding wireforms or stents supporting the fabric of the device generally confer a shape memory for the entire graft device to form a straight, cylindrical tube, which may conform well to vessel segments which are also straight and cylindrical in shape, but which will tend to act as a "round peg in a square hole" in regions of vascular anatomy that have irregular vessel shape, diameter or angulation. This is particularly so for the curved segments of thoracic aortic arch, and for the curved or angulated/tortuous neck region of abdominal aneurysms. Typically the graft lies obliquely or haphazardly in these segments and does not achieve successful attachment or seal.

WO 00/76423 A1 describes a bifurcated aortic graft comprising ipsilateral and contralateral legs. The graft comprises a combination of balloon expandable and self-expanding wires. The self-expanding wires increase the anchoring force between the bifurcated graft and the modular extension grafts.

US 2003/0105516 A1 describes a stent comprising a plurality of segments including a first and second balloon expandable segment, and a first self-expanding segment disposed between the first and second balloon expandable segments.

WO 03/082152 A1 describes a stent comprisng a self-expandable first member and a second member capable of expanding with the application of an external force. The first and second member are joined by a connector.

WO 03/063729 A2 describes an intraluminal endoprosthesis device comprising a tubular shaped balloon expandable stent and a self-expanding flared end.

### Summary of the Invention

The present invention consists in an intraluminal device comprising a tubular main body extending from a first end to a second end, said main body having a plurality of expandable wireforms; wherein the wireforms are grouped in a first group of one or more self expandable wireforms and a second group of one or more pressure expandable wireforms and wherein the main body comprises alternating groups of wireforms comprising said first group and said second group.

In one embodiment, both the first group and the second group of wireforms may each comprise a single wireform. Alternatively, the first group and the second group of wireforms may each comprise a plurality of wireforms.

The number of wireforms in the first group may be the same as the number of wireforms in the second group. Alternatively, the number of wireforms in the first group may differ from the number of wireforms in the second group.

The tubular main body comprises a plurality of said first group of wireforms and a plurality of said second group of wireforms. However, it is envisaged that the tubular main body may also comprise either one of said first group or one of said second group. In this embodiment, if the main body comprises only one of said first group, it will comprise more than one of said second group. Alternatively, if the main body comprises only one of said second group, it will comprise more than one of said first group.

In the embodiment wherein the tubular main body comprises a plurality of said first group of wireforms, the number of wireforms in each first group may be the same. Alternatively, the number of wireforms in each first group may differ relative to each other.

Where the tubular main body comprises a plurality of said second group, the number of wireforms in each group may be the same. Alternatively, the number of wireforms in each second group may differ relative to each other.

The ratio of the first group to the second group of the main body may be 1:1. In this embodiment, the groups may alternate in a "uniform" sequence ie 1;2;1;2;1;2 etc. (with 1 representing a first group and 2 representing a second group) along a length of the tubular main body.

Alternatively although having a ratio of 1:1, the first and second groups may alternate in a "non-uniform" sequence. For example, it is within the scope of this invention that two first groups are followed by one second group followed by a first group followed by two second groups etc. ie 1;1;2;1;2;2 etc. Other combinations of groups are envisaged and are considered to constitute part of the invention.

If the main body comprises more than one first group, each or at least some of the first groups may differ from each other. For example, the wireforms of the groups may be made from a different material. Furthermore, the wireforms may vary in configuration. For example, one first group comprising a sinusoidal configured wireform and at least a second first group comprising a "zig-zag" configured wireform.

Similarly, the second groups may differ from each other. Particularly, the wireforms of the plurality of second groups may differ in material and/or configuration.

The ratio of the first group to the second group may be between 1:20 and 20:1. As discussed above, the sequence of the groups of wireforms may be "uniform" or "non-uniform".

The wireforms of either of the first or second group typically extend circumferentially around the tubular main body. The wireforms may be woven into the material of the tubular main body. Alternatively, the wireforms may be tied to the tubular main body or secured using suitable biocompatible adhesives.

The wireforms are typically radially compressible and expandable such that the tubular main body is moveable between an insertion diameter, in which state the device may be inserted intraluminally into a vessel and a larger, expanded diameter in which state the device may be secured within the vessel.

In the expanded state, the diameter of the tubular main body corresponds generally to the diameter of the vessel in which it is positioned, for example, an aorta. In this preferred embodiment, the diameter of the main body may be configured to be a variety of sizes, one of which is selected according to the size of the vessel of the patient into which the device is to be implanted. When the device is used to bridge an aortic aneurysm it is preferred that the tubular main body has an expansion diameter of between 18 and 34 mm for the abdominal aorta, and between 20 and 46 mm for the thoracic aorta.

The tubular main body of the device may be linear along its length. Alternatively, the main body may bifurcate at a septum region into two leg portions. The latter embodiment may be particularly desired when bridging an aneurysm that extends across a natural bifurcation of the vessels in a patient.

The tubular main body comprises at least one protrusion portion which has a greater diameter than the remainder of the tubular body at least when said tubular body is in the expanded state.

The tubular main body may be made of a tube of woven polyester fabric. Other materials that may be desirable include expanded polytetrafluoroethylene (ePTFE), coated polyester, porous polyurethane, silicone, and spun or woven polymeric fibers. One of skill in the art of biocompatible intraluminal devices will readily identify other materials suitable for application in the construction of the tubular main body. It is preferred that the tubular main body be made of a material which is porous, thereby allowing tissue ingrowth into the material and/or formation of an intimal layer, although for some applications it may be desirable to make the tubular main body of a fluid impervious material.

Preferably, the fabric is woven into the tubular configuration of the main body, thereby eliminating seams or other internal protrusions which could interfere with blood flow or form locations for thrombi to occur.

The tubular body may comprise a flexible fabric such that it readily folds to accommodate radial contraction of the device, such as is necessary for intraluminal introduction of the device.

The tubular main body may be made from one material or from a plurality of different materials. The at least one protrusion portion of the tubular main body is made from a relatively more flexible material than the material of the remainder of the main body. Said portions typically expand to a larger diameter than the diameter of the remainder of the main body when the tubular main body is in said expanded state.

Alternatively, said at least one protrusion portion may be made from the same material as the remainder of the main body but wherein the weave of the fabric differs to provide a portion which expands to a larger diameter than the remainder of the main body when in its expanded state.

At least one balloon expandable wireform extends circumferentially around at least one protrusion portion of the tubular main body. Thus, when the device is deployed and the balloon expandable wireform is radially extended by inflation of a balloon, the feature that said portions have a greater diameter than the remainder of the main body provides one or more regions of enhanced device/vessel wall apposition.

Examples of clinical situations where the embodiment having a protrusion portion would be particularly advantageous include:
(i) Deployment of a thoracic endograft into the thoracic arch of aorta or curved segments of the upper descending aorta.

In these sites, a conventional self-expanding graft will typically be deployed as a regular cylinder in shape, so that one side of the graft may be pushed excessively against the inner curve of aortic wall (with regions of non-apposition above and below the segment of contact), whereas the straight opposite side of graft will be directed towards the outer curve of the angulated vessel, again failing to attach or connect over a significant length and potentially allowing dislodgement of the graft from the vessel wall, or leakage of blood-flow around this site.

The device of the present invention comprises one or more protrusion portions which correspond with these irregular regions of aortic anatomy, wherein said one or more protrusion portions are supported by one or more balloon-expandable wireforms, which will allow that section of the main body to be expanded outwards into good contact and conformability with the irregular vessel wall.
(ii) Deployment of an abdominal endograft into an irregular-shaped or angulated/curved region of infrarenal aortic neck, associated with an abdominal aortic aneurysm.

In these sites, a conventional self-expanding graft will typically be deployed as a regular cylinder in shape, so that one side of the graft may be pushed excessively against the inner curve of aortic wall (with regions of non-apposition above and below the segment of contact), whereas the straight opposite side of graft will be directed towards the outer curve of the angulated aortic neck, again failing to attach or connect over a significant length and potentially allowing dislodgement of the graft from the vessel wall, or leakage of blood-flow around this site.

The device of the present invention has at least one protrusion portion comprising an enlarged, first or second end of the main body. Said at least one protrusion portion may correspond to the abovementioned irregular regions of aortic anatomy. The at least one protrusion portion is supported by one or more balloon-expandable wireforms, which will allow that section of the main body to be expanded outwards into good contact and conformability with the irregular vessel wall. The enlarged end(s) of the main body may comprise a trumpet-shaped end. Said end may be reinforced by pressure-expandable wireform(s) and may be forced out into reliable contact and conformability with the aortic wall, to enhance seal of blood-flow and efficient attachment of the graft to the aortic wall.

The pressure expandable wireforms are balloon expandable wireforms, but may include an element of self-expanding, spring material within their circumference.

The balloon expandable wireforms of the present invention are preferably made of an alloy of carbon, silicon, phosphorus, sulphur, chromium, nickel, beryllium, cobalt, iron, manganese and molybdenum which is sold under the Elgiloy trade mark. Other materials which may be utilized in making the wireforms include a nickel and titanium alloy sold under the Nitinol trade name; stainless steel, and other biocompatible metals.

Preferably, each of the balloon-expandable wireforms has a curvilinear geometry including a closed sinusoidal-like wave geometry with alternating crests and valleys. The balloon-expandable wireforms are preferably secured to the material of the tubular main body by weaving the wireform through the fabric material. Alternatively, the wireforms may be secured by suture, glues, and other methods. The wire may be woven through the fabric such that a distal tip of the valleys of each wireform extends through the graft and is positioned on the outside of the fabric structure.

In another embodiment, the balloon expandable wireforms are a zig-zag configuration with more angular crests and valleys.

The balloon expandable and/or self expanding wireforms may extend around the entire circumference of the tubular main body. Alternatively, the wireforms may extend around a portion of the circumference of the main body. In a further embodiment, the wireforms form a continuous structure around the circumference of the main body. Alternatively, the wireforms comprise intermittent members having a series of gaps between a series of main members. Such a structure generally comprises a sinusoidal or zig-zag configuration.

The configuration of each of the self-expanding wireforms is typically naturally biased towards an expanded state. The self-expanding wireforms may be made of the same or a different material to the balloon expandable wireforms. An example of a suitable material is an alloy of nickel and titanium. A further example includes an alloy of carbon, silicon, phosphorus, sulphur, chromium, nickel, beryllium, cobalt, iron, manganese and molybdenum. While the same base material that is used in the construction of the balloon-expandable wireforms may be used for the self expanding wireforms, the method of manufacturing would differ.

The self-expanding wireforms may have a generally curvilinear configuration having waves which define crests and valleys. Preferably, the self expanding wireforms have a sinusoidal configuration. Alternatively, the self expanding wireforms may be "S or have a zig-zag configuration.

Preferably each of the self-expanding wireforms is positioned on an exterior surface of the tubular main body.

Preferably the wireforms, both self expanding and balloon expandable, are positioned in a spaced apart manner such that the they do not interfere with each other in either a radially expanded or contracted state. In a further embodiment, the wireforms are closely spaced.

In a preferred embodiment the valleys of one wireform are aligned with the crests of an adjacent wireform. Alternatively, the crests of one wireform may be aligned with the crests of an adjacent wireform.

In a further embodiment, adjacent wireforms may be attached to one another. Particularly, an adjacent self expanding wireform and balloon expandable wireform may be connected to each other at one or more regions by one or more strut members. Typically, a crest or valley may be connected to a respective crest or valley of an adjacent wireform by a strut member. The strut members of this embodiment may connect all the crests of one wireform to all the crests of an adjacent wireform. Alternatively, only some of the crests of one wireform may be connected to corresponding crests of adjacent wireforms. The struts may further connect a valley of one wireform to a crest of an adjacent wireform. All the crests of one wireform may be connected to all the valleys of an adjacent wireform by said struts.

In a further embodiment, rather than connect to each other, adjacent wireforms may be positioned in close proximity relative to one another. This allows the movement of each wireform to have an effect on a common area of the main body.

A wireform may include one or more strut members wherein said strut member(s) extend from a region of the wireform but do not engage with an adjacent wireform of the tubular main body. Particularly, these struts may extend from a crest or a valley of the wireform.

The configuration of one wireform may differ from the configuration of an adjacent wireform. For example, one wireform may be sinusoidal in configuration whereas the adjacent wireform has a zig-zag configuration.

The device of the present invention, having both balloon-expandable and self expanding wireforms, enables precision in placement of the device. The self-expanding wires may open within the vessel immediately upon deployment by techniques of sheath retraction to expose the device from its constraining sheath or catheter, which then allows insertion or overlap of other deployment catheters, balloon catheters or modular components. Expansion of these spring elements also opens a wider internal path for deployment catheters, sheaths or the inflatable balloons and particularly any which were pre-loaded in the process of device preparation and packaging. The self expanding wireforms also provide radial elasticity or crush resistance in addition to flexibility. The balloon expandable wireforms provide the tubular main body with a greater radial force and hoop strength and a more predictable degree of recoil, thereby conferring a property of allowing true moulding of the device outer wall to the contours of the treated vessel wall.

The feature of the invention that balloon expandable wireforms are positioned between self expanding wireforms and vice versa is particularly advantageous. Particularly, it is a preferred feature of this invention that there are sufficient self expanding wireforms having sufficient expansion force to cause one or more of the balloon expandable wireforms to at least partially move from a radially compressed state towards a radially expanded state. This may be particularly advantageous in regions of graft attachment or fixation to the vessel wall, or to an overlapped graft segment, such as those used in modular or extension graft techniques.

The balloon expandable wireforms may comprise a sinusoidal shape with crests and valleys. The balloon expandable wireforms may have a varying thickness along their length with some portions of the wireform thicker than other portions. In one embodiment, at least part of the crest portions and/or at least part of the valley portions may be thinner than the remainder of the wireform. This particular embodiment provides a balloon expandable wireform that has reduced resistance to an outwardly directed force in an initial compressed state. The configuration of this embodiment provides a device wherein when deployed has self expanding wireforms that move from their compressed to their expanded state and in doing so cause the balloon expandable wireforms to also move at least partially towards their expanded state. The full expansion of said balloon expandable wireforms may be achieved by using an inflatable balloon or other mechanical means.

In the above embodiment. because the balloon expandable wireforms are at least partially expanded by the self expanding wireforms, less pressure is required to force the balloon expandable wireforms to their fully expandable state. A smaller diameter balloon may thus be used to first "open" the wireforms towards their expansion diameter, providing a more compact assembly for the introduction of the device into a vessel. This initial small-diameter balloon may be pre-loaded within the graft lumen at the time of packaging or preparation, prior to insertion into the vessel. Subsequent larger-diameter balloons can then be sequentially introduced and inflated to increase the diameter of the graft device.

Further, in another embodiment, the delivery system used to deploy the intraluminal device need not contain a balloon at all, thereby significantly reducing the bulk of the delivery system. The self expanding wireforms may be sufficient to open the balloon expandable wireforms to create a flow through lumen. A separate balloon may subsequently be introduced as a separate step to fully expand the balloon expandable wireforms.

In a further embodiment, expansion of the balloon expandable wireforms may cause at least one or more self expanding wireforms to move into a radially expanded configuration. Particularly, when the main body is deployed in a vessel of a patient and the self expanding wireforms "spring" open, the subsequent expansion of the balloon expandable wireforms may cause the self expanding wireforms to radially extend further, bringing certain regions of the main body into tight engagement with the vessel wall. The flexible or relatively malleable nature of, or weak recoil of some self expanding wireforms allows such "over-expansion", especially when held open by the force of the expanded pressure-expandable wireform, which may be particularly useful when trying to achieve a secure fit within an abnormally shaped vessel.

In turn, the balloon expandable wireforms when deployed preferably have sufficient hoop strength to counter any recoil of the self expanding wireforms.

The intraluminal device of the present invention may be used to tailor a device suitable for any number of different configurations of a vessel. For example, the neck of abdominal aorta may be shortened and very angled due to the position and size of an aortic aneurysm. In such cases it is difficult to secure an upstream distal end of an intraluminal device in this angled region. Often a gap is left between a region adjacent the distal end and the vessel wall. In one embodiment of this invention, the main body may comprise a balloon expandable wireform at its distal end and one or more self expanding wireforms adjacent to said balloon expandable wireform. The balloon expandable wireform may be extended to engage with the vessel wall. Alternatively, the first wireform may be self-expanding and the second balloon-expandable. The balloon expansion of the balloon expandable wireform alone or further expansion of the self expanding wireforms by the balloon may cause the device in this region to "over-extend" and to be brought into tight engagement with the angled vessel wall.

Also, within the thoracic aorta, angulated or larger diameter regions of aorta, particularly in or near the aortic arch, may be better treated by this device and method. Similarly, if an aneurysm causes a highly extended region between two narrower regions, balloon expandable wireforms may be utilised to provide a tight engagement with said narrower regions and one or more self expanding wireforms may be utilised to extend into the highly extended region of the vessel.

The tubular main body may further include at least one radiopaque marker to assist in the placement of the device.

In a second aspect, an intraluminal device for positioning within a branched vessel of a patient not part of the present invention is disclosed, said device comprises an elongate main body and at least one branch portion branching therefrom, wherein said branch portion of the intraluminal device is independently moveable relative to the main body.

In a third aspect, a steering mechanism for a branch portion of a branched intraluminal device not part of the present invention is disclosed, said steering mechanism comprises a catheter having a first elongate tubular member and at least one second elongate tubular member which extends from a proximal end to a distal, manipulable tip, said tip being moveable in two or more planes relative to said first elongate tubular member.

The steering mechanism of the third aspect may be used to steer the branch portion of the intraluminal device of the second aspect. In this embodiment, the catheter may be pre-loaded into said intraluminal device. Alternatively, the catheter may be introduced through the intraluminal device during a procedure.

In a fourth aspect, an intraluminal assembly not part of the present invention is disclosed it comprises: an intraluminal device having an elongate main body and at least one branch portion branching therefrom;
said main body defining an internal lumen to receive a catheter, said catheter comprising a first elongate tubular member and at least one second elongate tubular member;
wherein said second elongate tubular member extends from a proximal end to a distal, manipulable tip, said manipulable tip insertable into said at least one branch portion to move said at least one branch portion relative to the main body.

In a fifth aspect, a method of positioning an intraluminal device within a branched vessel of a patient not part of the present invention is disclosed, the intraluminal device comprises a main body and a branch portion, the branched vessel comprising a pre-branch vessel and at least one post-branch vessel, said method including:
(i) introducing the intraluminal device in a collapsed configuration to a position within the pre-branch vessel;
(ii) causing the branch portion of the device to move independently of the main body to position said branch portion within a post branch vessel; and
(iii) causing or allowing the intraluminal device to expand such that at least a length of the branch portion is secured within the post-branch vessel.

The branch portion of the intraluminal device may comprise a flexible member. The branch portion may be relatively more flexible than the main body of the device. In this embodiment, the branch portion may be made from a different material to that of the main body of the intraluminal device.

The branch portion may be less reinforced than the main body. For example, the intraluminal device may comprise a graft which is reinforced along it length by a series of wireforms. The wireforms may be more closely spaced in the main body than the wireforms in the branch portion. The wireforms of the main body may be thicker than those of the branch portion. Alternatively, or in addition, the wireforms of the main body may be made from a different material to those of the branch portion.

The graft material of the branch portion may comprise a polyurethane material or, alternatively PTFE.

The intraluminal device may further comprise a stent wherein the main body may differ in the structure of the stent cells to the structure of the cells in the branch portion. The material of the stent of the main body may also differ from the material of the branch portion.

The flexibility of the branch portion enables a user to manipulate its orientation during use to properly secure a branched graft or stent within a branched vessel as will be described in more detail below.

The branch portion moves relative to the main body in a number of orientations. The movement of the branch portion may be caused by manipulation of a steering mechanism of the branch portion itself.

For example, the intraluminal device may comprise an elongate steering member which is releasably attached to the branch portion. The steering member may comprise an elongate wireform of suitable rigidity to cause the relatively flexible branch portion to move in response to movement of said wire. The elongate wireform may be manipulated by the surgeon from control ends external the patient's body. The wireform may be connected to an internal or an external wall of the branch portion. Alternatively, the wireform may weave through the material of the wall of the branch portion. Actuation of the control ends by the surgeon may move the branch portion accordingly. The surgeon may monitor such manipulation of the branch portion using imaging techniques.

As described above, the branch portion may be moved by the second elongate tubular member of the catheter of the third aspect. In this embodiment, once the branch portion is in position within the vessel, the second elongate tubular member may be withdrawn.

Alternatively, the manipulable tip of the second elongate tubular member may be attached to or form part of the branch portion of the intraluminal device. The manipulable tip may be releasably attached to the remainder of the second elongate tubular member such that once the branch portion is positioned appropriately, the manipulable tip is released and left *in situ* to form an inner sheath of the branch portion of the device.

Both the first and the second elongate tubular members of the catheter may define an internal lumen along their length to receive a guidewire. The first elongate tubular member typically receives a primary guidewire to enable placement of the device within the target site of the vessel.

The guidewire of the second elongate tubular member extends beyond the second member and into the branch portion of the device. This guidewire may be further fed along the post branch vessel during use to allow connection of a further tubular extension to the branch portion of the device.

Following introduction of the branch portion of the intraluminal device into the branched vessel, the branch portion is caused or allowed to move from its collapsed configuration to its expanded configuration.

Expansion of the branch portion may be achieved by self expansion of the branch portion or be pressure expansion such as balloon expansion. The types of expansion is detailed above in relation to the first aspect of the invention.

The branch portion of the device is typically expanded prior to expansion of the main body of the device. Alternatively, the main body may be expanded before expansion of the branch portion. In another embodiment, the main body and branch portion are expanded at approximately the same time.

The control of the branch portion of the device allows a user to properly seat the bifurcation region of the device correctly ie in close contact with the bifurcation region of the vessel within which it is deployed. Known devices typically sit above the bifurcation region of a vessel and thus are more prone to leakage and slippage.

This device and method of these aspects represents a significant improvement over conventional techniques, in which a secondary branch or extension graft device is positioned through an opening of a primary trunk graft by passage over a guidewire or sheath into the designated branch and which if mis-positioned can block an artery. Using the device and method of these aspects of the invention, a secure access to the branch vessel may be achieved and confirmed before implantation (expansion) of the main body of the device.

The device and method may be used to introduce an intraluminal device into various branched vessels within the body including but not limited to: the branches of the aorta or iliac arteries within the chest, abdomen or pelvis, including branches of the aortic arch, including brachiocephalic, carotid or subclavian branches; or of the abdominal aorta including the coeliac, mesenteric, renal or iliac branches or of the iliac artery including the internal iliac branch.

The branch portion of the device once *in situ* may be secondarily connected (by methods such as modular overlap) to other modular endovascular graft/stent segments, such as an aortic endovascular graft as described above in relation to the first aspect.

The device and method may enable total re-lining of a prior-implanted endovascular graft/stent which has failed due to fabric degradation, migration, or other defects. In this application, the branch portion of the graft may initially be manipulated into the contralateral limb of the prior bifurcated graft, and the septum region of the replacement graft positioned in close proximity to the septum of the prior graft, permitting good conformation of the replacement graft inside the prior failed graft.

Because the branch portion of the graft is deployed into the designated branch vessel before completion of the deployment of the main body of the graft (which lies in the pre-branch vessel), the patency and sealing of the branch vessel can be assured early in the procedure, and any secondary grafts or extensions then have low risk of occluding the branch. Compared to the known techniques of side-branch grafting, by late, secondary placement of a tubular extension limb graft, there is a significant danger of prior blockage of the branch vessel by inadvertent misplacement of the primary trunk graft with part of its fabric covering the branch opening, due to misalignment or longitudinal movements, rotational twisting, etc. The prior branch devices feature a very short branch or simple opening (or hole) in the graft wall which are intended and designed to remain fully within the pre-branch vessel, without extending into the branch vessel, and act solely as a window or passage from the trunk graft towards the branch vessel (for overlap extension of the secondary tubular limb branch graft), but not as an implant or lining for that branch vessel.

When used for iliac artery branches, the branch portion of the graft is configured to be implanted from an ipsilateral femoral artery approach, giving further advantage over known techniques which require implant of an iliac branch tubular extension from either a contralateral femoral approach (by cross-over sheath over the aortic bifurcation), or from an upper limb, both of which have significant disadvantages and in many cases are not possible for certain patients who have unsuitable anatomy, or who have had prior vascular surgery procedures.

The introduction and deployment of the graft and particularly the branch portion into the post-branch vessel may involve the step of positioning a guidewire into the target post-branch vessel and using the guidewire as a guide for the subsequent passage of the second elongate tubular member of the catheter and the branch portion of the graft.

### Brief Description of the Drawings

Figures 1 to 5 and 7 to 20 do not show embodiments of the present invention. Figure 1 is a schematic view of an intraluminal device,
Figure 2 is also a schematic representation of an intraluminal device ;
Figure 3 is a schematic representation of the device when implanted within an artery of a patient;
Figure 4 depicts a further the device when implanted within an artery of a patient;
Figures 5a to 5i are schematic representations of various devices, showing various combinations and types of wireforms;
Figure 6 is an embodiment of the device of the present invention showing protrusion regions on the main body of the device;
Figure 7 shows a still further schematic representation of a device;
Figure 7a is a schematic representation of a prior art device in a neck of aorta;
Figure 7b shows the device in a neck of aorta;
Figure 8 depicts a device;
Figures 9a and 9b depict a method and device for positioning a side branch particularly a directional catheter/guidewire pre-loaded inside a branched graft;
Figures 10a, 10b and 10c depict a double-lumen directional catheter for use with the method and device;
Figures 11a to 11d depict a branched graft demonstrating the various angles that the branch portion can make with respect to the main body of the graft, as directed by the catheter component and guidewires (not shown);
Figures 12a and 12b depict pre-curved branched grafts with longer side portions;
Figures 13a and 13b depict primary branched grafts with longer side portions;
Figures 14a to 14d depict double layer graft techniques; with overlap of a reinforcing graft within the main body of the graft;
Figure 15 depicts a device positioned in a side branch; the branch portion in the internal iliac branch and the main body of the graft component in the main iliac artery.
Figures 16a and 16b depict a device in application for treatment of an abdominal aortic aneurysm;
Figure 17 is a continuation of the deployment process shown in Fig 16,and depicts the side portion *in situ* within the branching vessel (right common iliac); and the overlap of other graft components to complete the sealing and exclusion of the aneurysm sac;
Figures 18a and 18b depict the initial steps of re-lining a failed endograft using a steerable side-branch device;
Figures 19a and 19b depict continuation of the total graft re-line of Fig.18b involving modular graft deployment;
Figures 20a to 20g depict steerable sleeve devices, of suitable shape and flexibility to form the elongate tubular member of the catheter.

### Description of the Preferred Embodiments

The device is generally depicted as 10 in the accompanying drawings. The device 10 comprises a graft 11 for bridging a site of a damaged vessel, such as an artery.

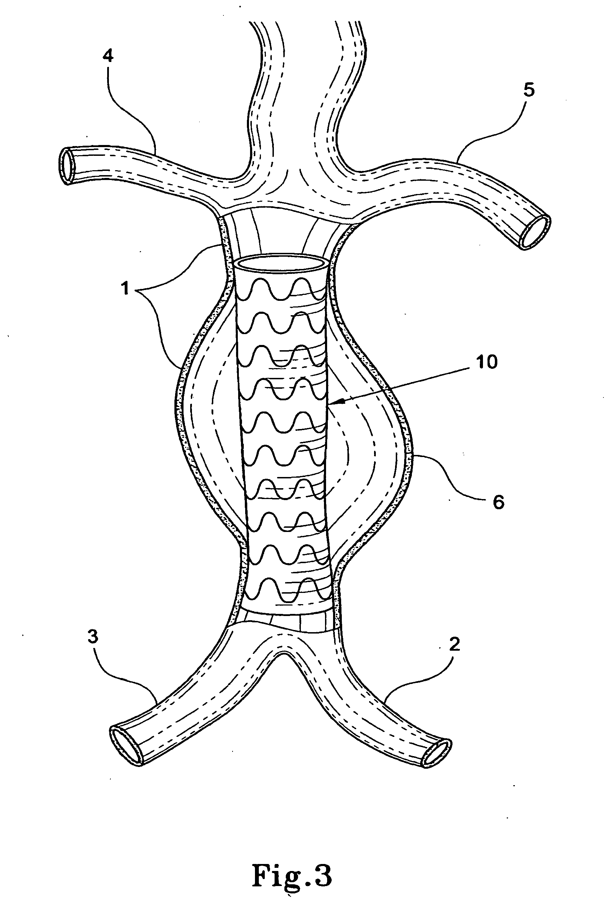

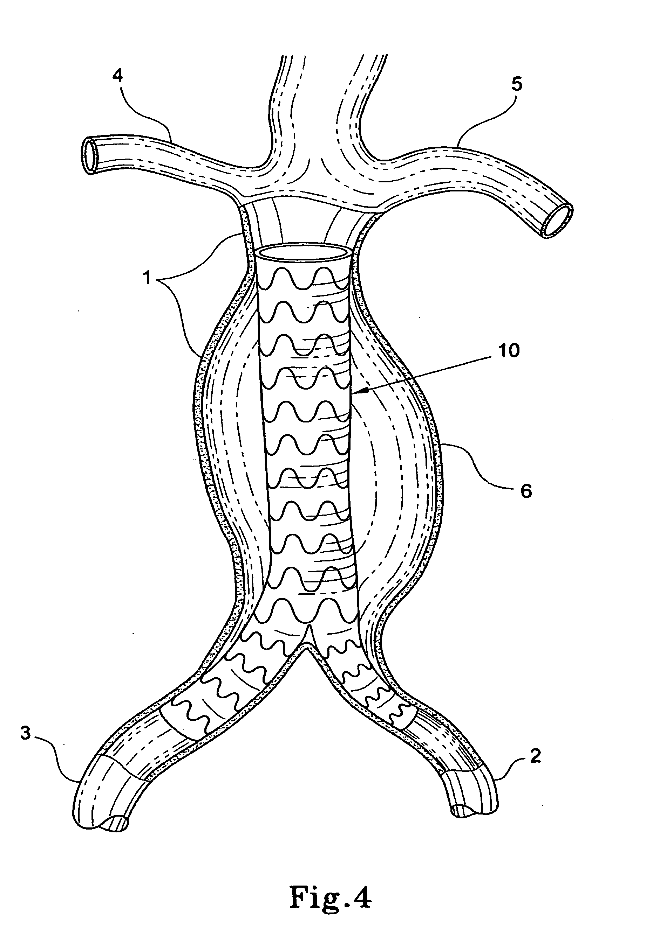

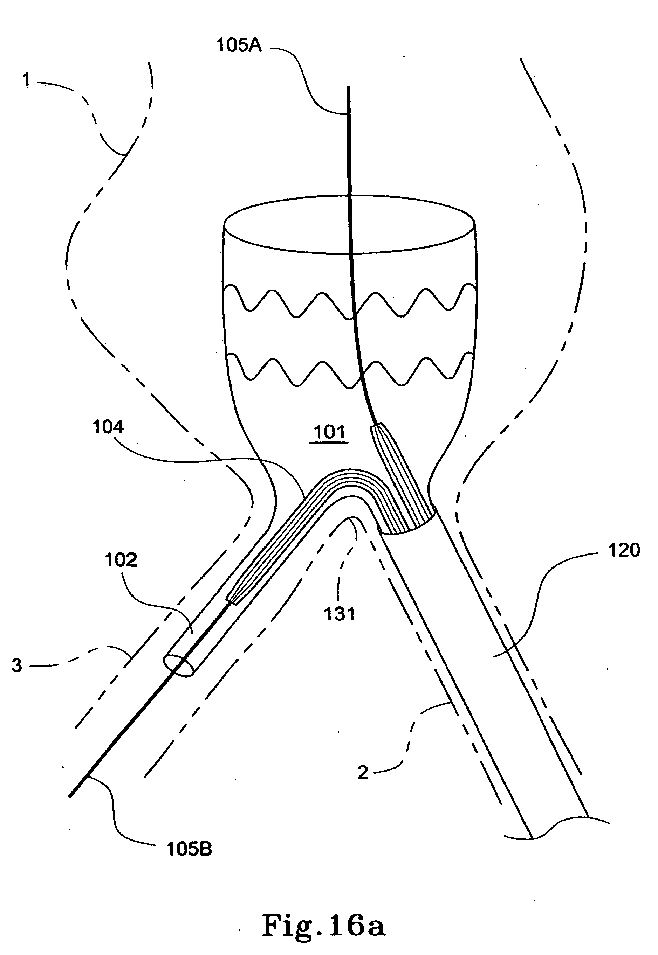

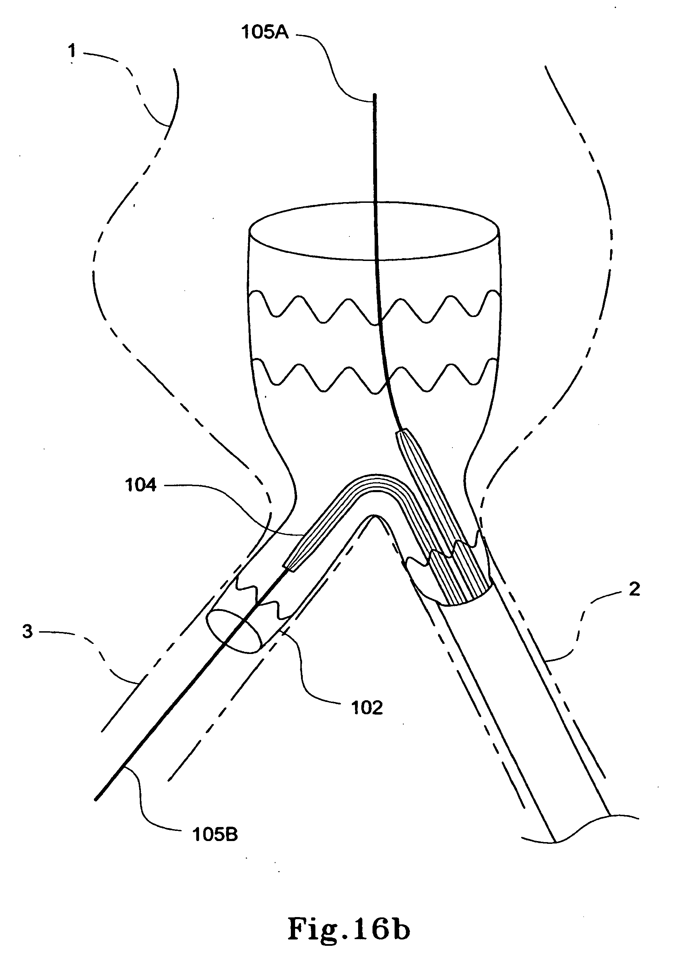

Aneurysms frequently form in the thoracic aorta, or in the abdominal aorta at a location between the renal arteries and immediately proximal to the common iliac arteries. FIGS. 3 and 4, for example, illustrate the anatomy of the abdomen in the location of an aortic aneurysm. The abdominal aorta 1 can be seen branching distally into the common iliac arteries 2, 3. The right and left renal arteries 4, 5 are located proximal to the common iliac arteries. Between the common iliacs and the renal arteries, an aortic aneurysm 6 is shown as a distended section of the abdominal aorta 1. Aneurysms also frequently form in the iliac arteries, between the aorta and the femoral arteries. FIGS. 16a and 16b, for example, illustrates the anatomy of an aneurysm of the left common iliac artery.

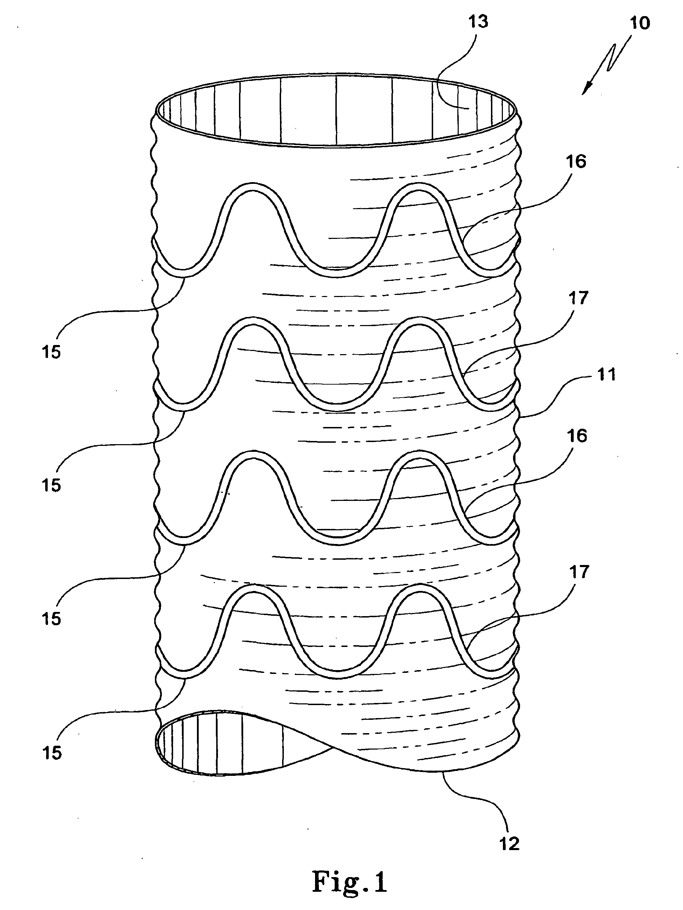

A graft is depicted in Figure 1. The graft 11 is configured from a flexible tubular main body 12 with a lumen 13 running the length thereof. The tubular main body 12 is reinforced by wireforms 15 extending circumferentially around the tubular structure.

The flexible tubular structure is foldable and the wireforms 15 are radially compressible and expandable. Therefore, the graft 11 is configured to move between an insertion diameter, in which state the graft may be introduced through a femoral and iliac artery to the site of placement in the aorta 1, and a larger, expanded diameter (as depicted in all the accompanying figures) in which state the graft may be secured within the aorta 1.

In the expanded state, the graft 11 is generally cylindrical and may be configured to a variety of sizes.

The graft depicted in Figure 3 is a simple tubular graft to bypass an aneurysm in the aorta. The graft 11 in Figure 4 is bifurcated to bridge an area of aneurysm that extends across the bifurcation of the aorta 1 to the common iliac arteries 2, 3. When used as a bifurcated graft, the general structure of the device may be a single-piece bifurcated design as depicted, or more typically may be a multi-piece modular device with several connected or overlapped components.

In accordance with a presently preferred embodiment of the invention, at least a first group of one or more self expanding wireforms 16 and a second group of one or more balloon expandable wireforms 17 are provided on the tubular main body 12. The main body 12 comprises alternating groups of wireforms comprising the first group and the second group.

Either the balloon expandable 17 or the self expanding wireforms 16 may take on a number of configurations relative to the tubular body: For example, as depicted in Figure 1, both types of wireforms are sinusoidal in configuration. Alternatively, either type of wireform 15 may have a zig-zag configuration. In each of these configurations, the wireform generally comprises a crest 18 and a valley 19.

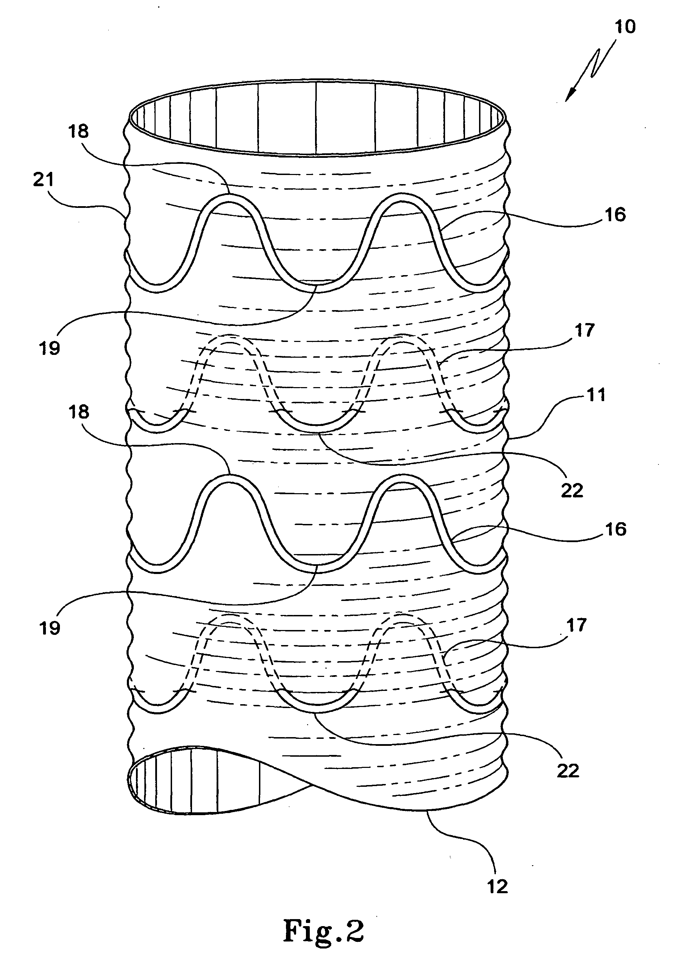

The wireforms 15 may be woven into the material of the tubular main body as depicted in Figure 2. Alternatively, the wireforms 15 are positioned on an exterior surface 21 of the main body 12. In Figure 2, the balloon expandable wireform 17 is woven into the fabric with a distal tip 22 of the valley 19 of each wireform extending through the graft and positioned on the outside of the fabric structure.

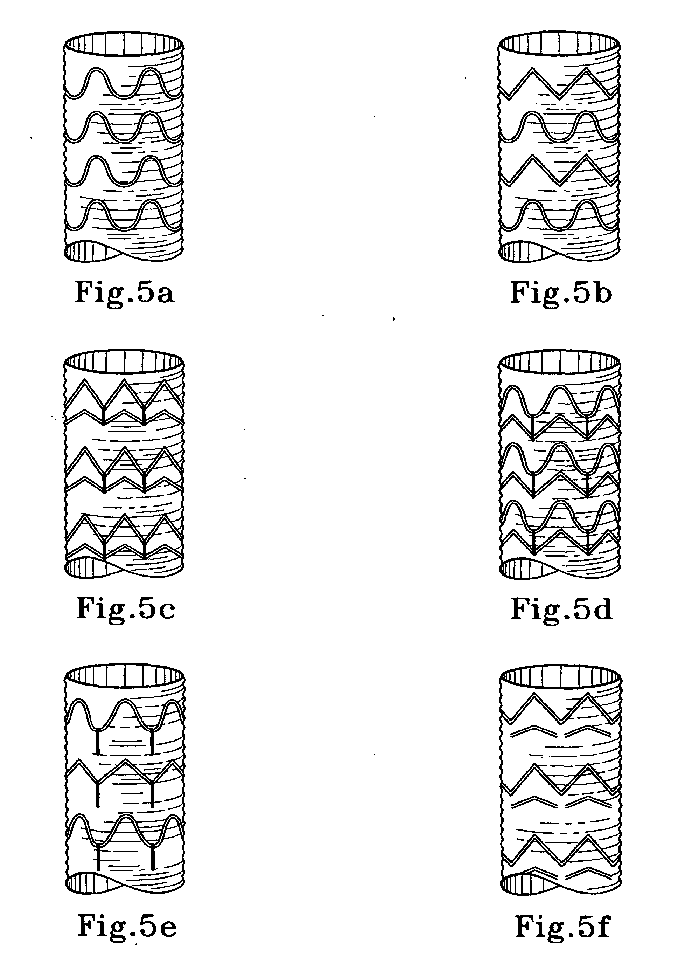

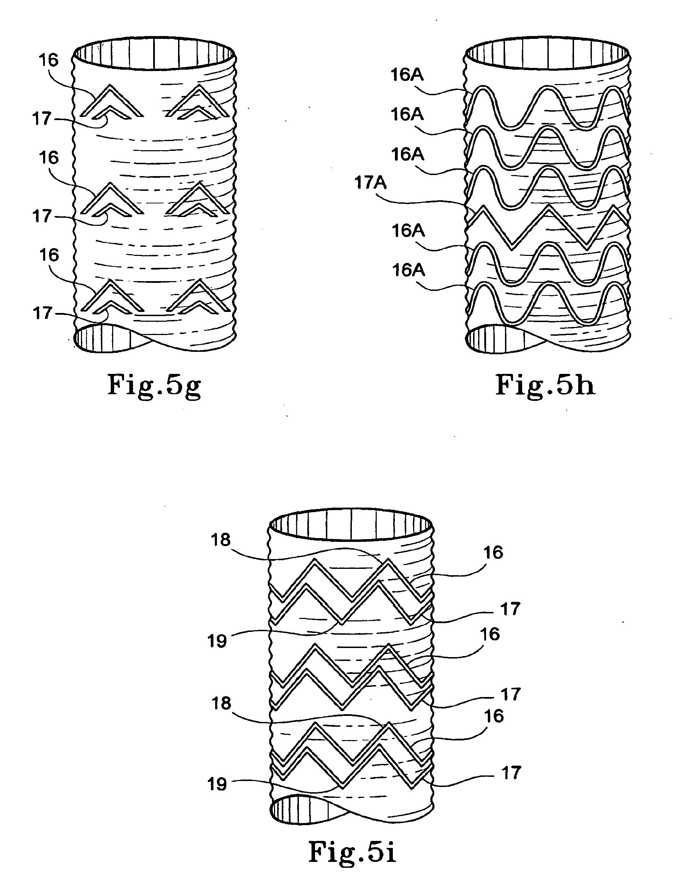

As shown in Figures 5a to 5i, the balloon expandable wireforms 17 extend around the entire circumference of the tubular main body 12. However, as depicted in Figure 5f, the balloon expandable wireforms 17 can comprises an intermittent structure having a series of gaps 23 between a series of main members 24.

The self-expanding wireforms 16 also have a generally sinusoidal configuration as shown in the embodiment in Figure 5a.

In Figures 5c and 5d, adjacent wireforms 15 are attached to one another. Adjacent self expanding and balloon expanding wireforms 16,17 are connected by struts 25 extending between valleys 19.

The struts 25 depicted in Figure 5e do not engage an adjacent wireform of the tubular main body.

The wireforms in Figure 5i are closely spaced such that there are areas of "overlap". While the wireforms do not touch each other there is a common region of the main body between crests 18 of one wireform and valleys 19 of an adjacent wireform.

The configuration of one wireform may vary from the configuration of an adjacent wireform. For example, one wireform may be sinusoidal in configuration whereas the adjacent wireform has a zig-zag configuration. The example depicted in Figure 5b has zig-zag shaped self expanding wireforms 16 and sinusoidal balloon expanding wireforms 17. In Figure 5d, the reverse applies.

With regard to the alternating sequence of wireforms, it should be noted that the groups need not follow one another in an ordered fashion ie one group of self expanding wireforms 16 followed by a group of balloon expandable wireforms 17, followed by another group of self expanding wireforms 16 although this is certainly an embodiment of the invention. In Figure 5h, the first group comprises a single self expanding wireform 16a. Thus in this embodiment, three first groups are provided followed by one second group comprising a single balloon expandable wireform 17b. The second, balloon expandable group is followed by two first groups each group comprising a single self expanding wireform 16a. The sequence is, therefore, 1;1;1;2;1;1 where 1 represents a first group and 2 represents a second group.

The balloon expandable wireforms may comprise a sinusoidal shape as mentioned above wherein crests 18 and valleys 19 are present along the length of the wireform. The balloon expandable wireforms may have a varying thickness along its length with thinner crest portions 26 or valley portion 27. This particular example provides a balloon expandable wireform that has reduced strength in an initial state such that it may be caused to move towards an expanded state by the expansion of the self expanding wireforms of the device.

Because the balloon expandable wireforms are at least partially expanded by the self expanding wireforms, less pressure is required to force the balloon expandable wireforms to their fully expandable state. A weaker balloon may thus be used to "open" the wireforms to their full expansion diameter. More preferably, no balloon is required in a delivery system and the balloon expandable wireforms may be expanded further by a balloon which is introduced as a secondary step ie upon removal of the delivery system used to deploy the intraluminal device.

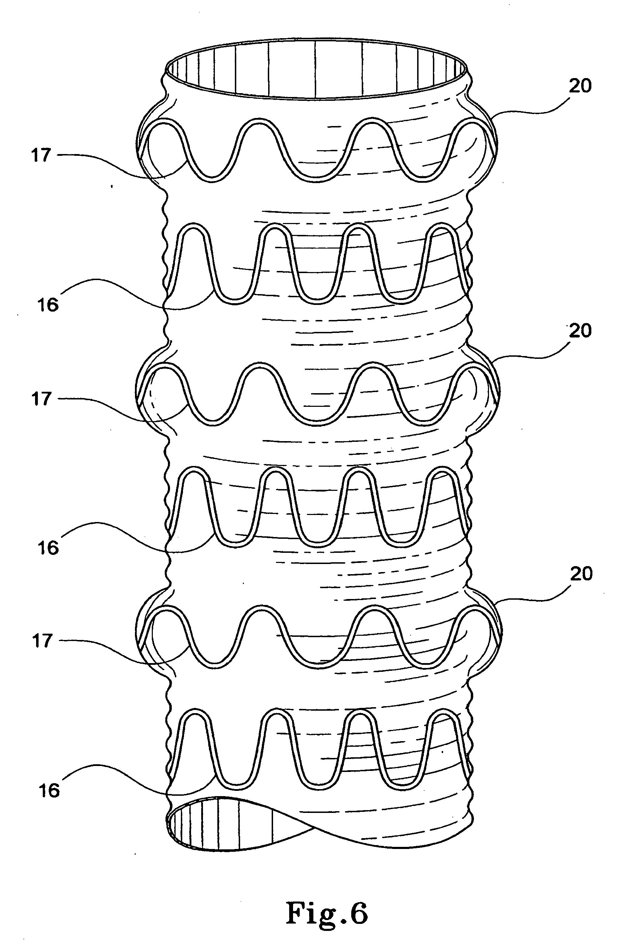

Figure 6 shows protrusion portions 20.

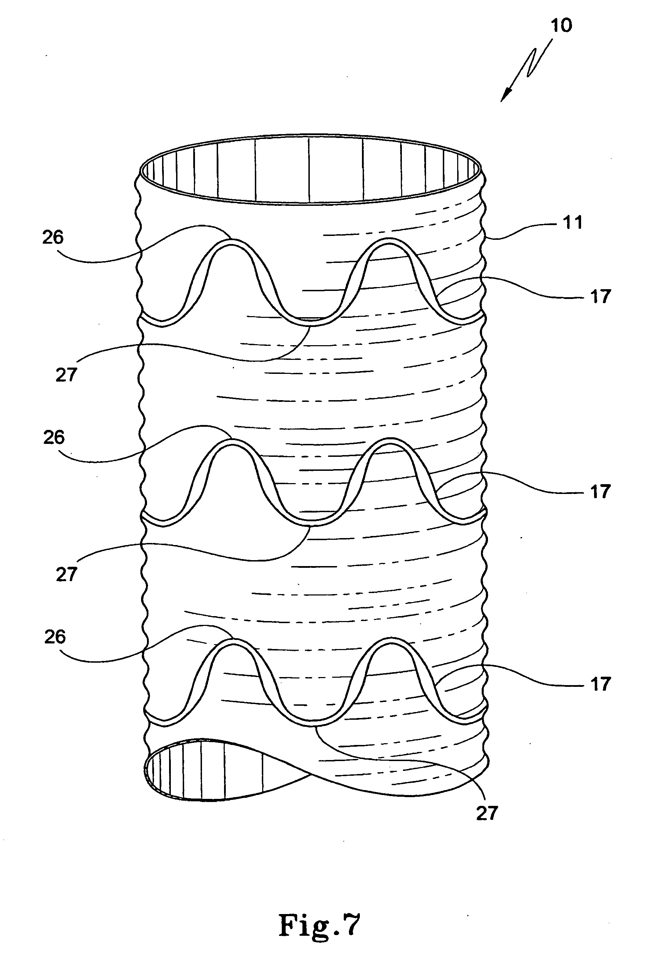

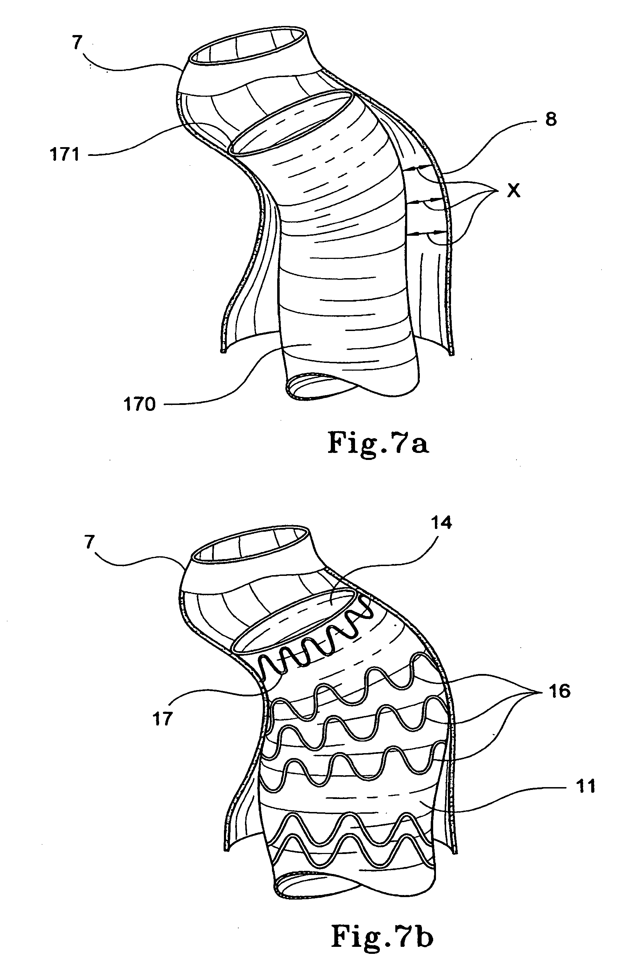

Figure 7a shows an abnormal, oblique neck 7 of aorta 1. Due to the angle formed by the neck of aorta 7, conventional devices as depicted as 170 do not provide a good fit. As seen, only the very distal end 171 of the device 170 is in engagement with the vessel. The region of the device adjacent to the distal end is spaced from the posterior wall 8 of the neck of aorta, leaving a gap (x) between the device and the wall.

The device depicted in Figure 7b has at least one balloon expandable wireform 17 at upstream end 14. The device has a series of self expanding wireforms 16 adjacent to the upstream end 14. When deployed in a vessel, the self expanding wireforms 16 "spring" open and the balloon expandable wireform 17 is caused to move to an expanded configuration wherein it engages the wall of the neck of aorta 7. The balloon may then be moved upstream such that it is positioned adjacent to the self expanding wireforms 16. Inflation of the balloon thus causes the flexible self expanding wireforms to move into an "over-extended" configuration to bring the device of this region into engagement with the vessel wall of the neck of aorta 7. The result is a far greater region of device/vessel wall apposition.

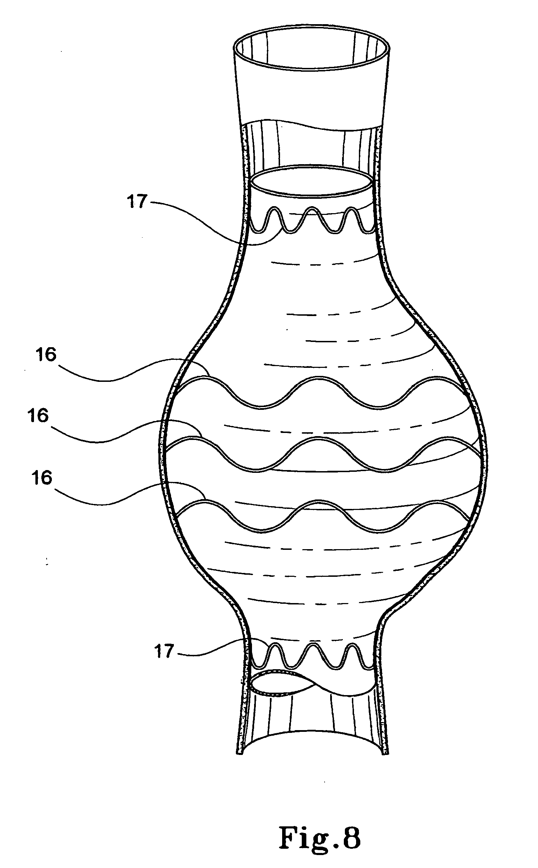

In Figure 8, the self expanding wireforms 16 allow the device to extend sufficiently to engage a distended region of a vessel.

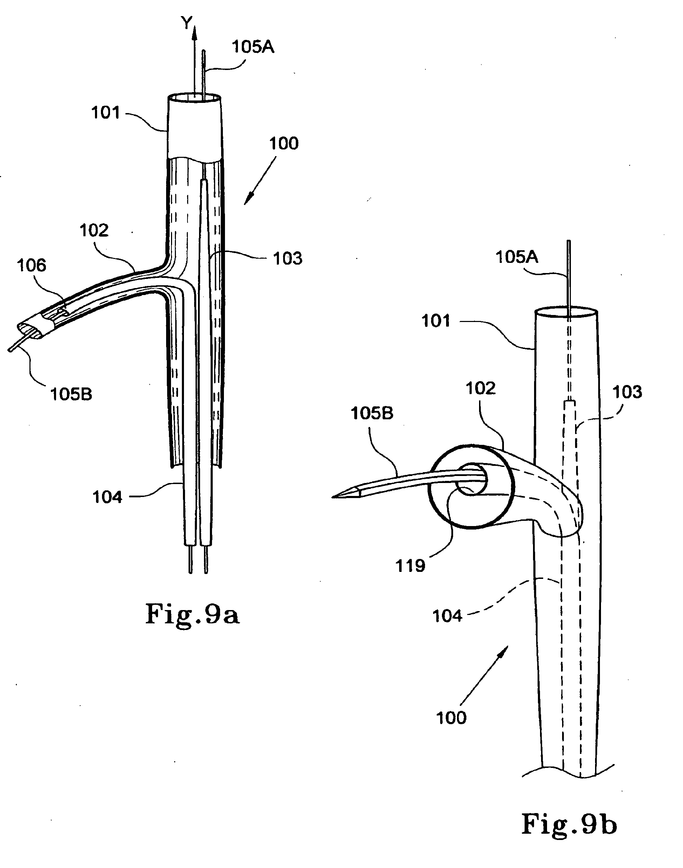

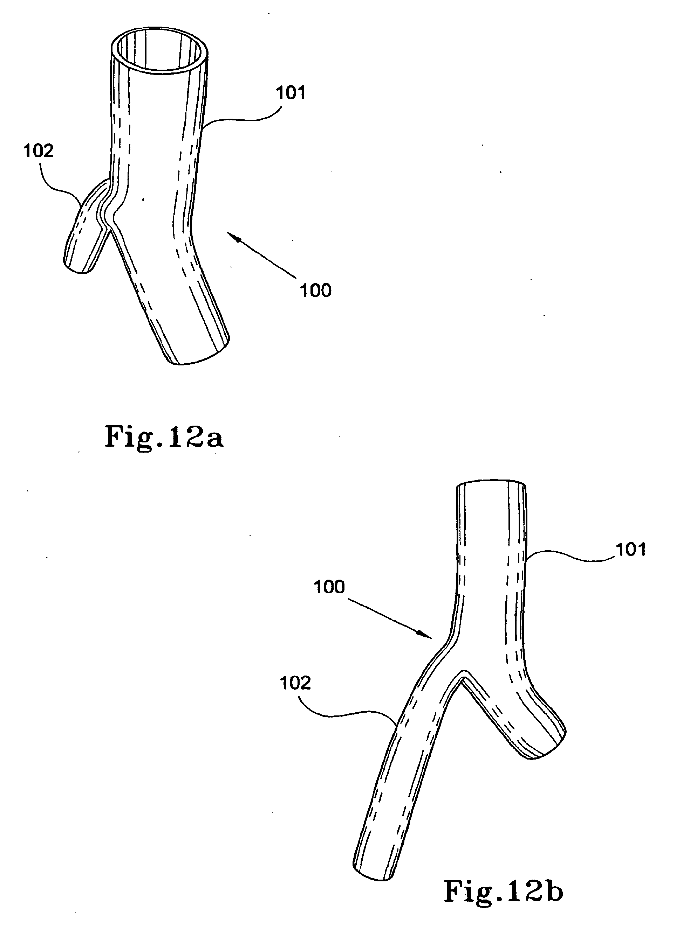

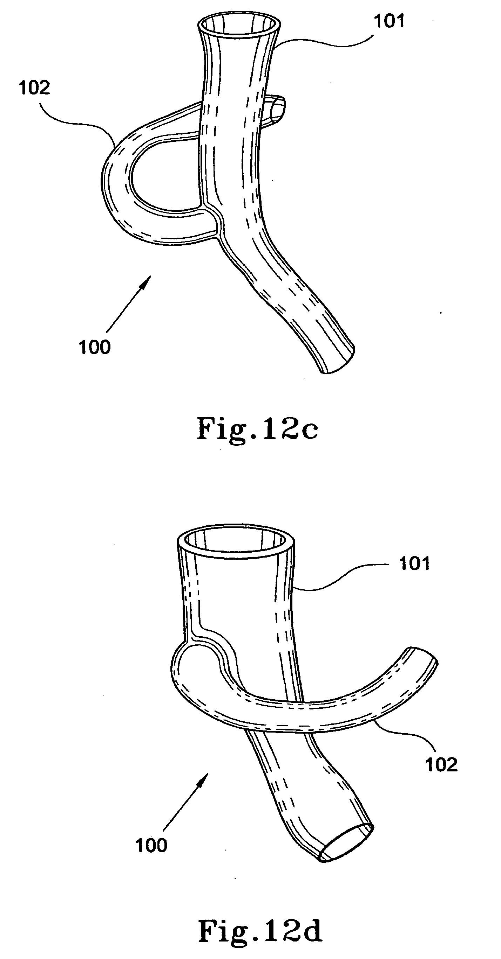

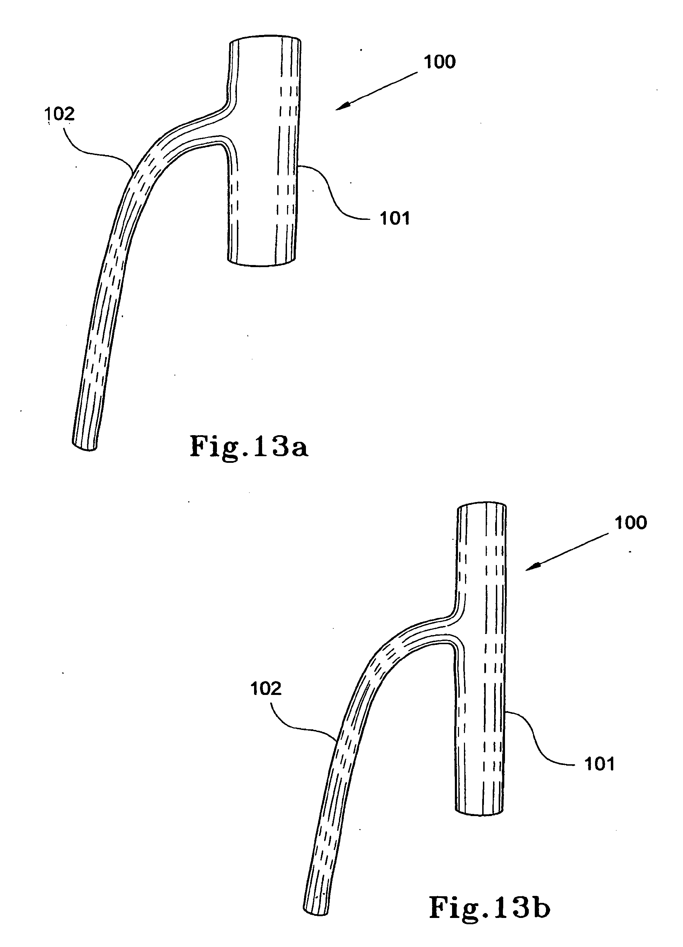

Figures 9 to 20 depict the use of directional branches of grafts/stents. The devices and methods of this further aspect provide a means for reliably grafting/stenting a branched vessel by allowing guidance of said branched component.

Known devices are aimed at bypassing areas at or around the iliac arteries only. The techniques are crude and depend on secondary cannulation of branches by wire, without any guidance technology.

The branched grafts and stents of this invention may use directional catheter technology such as described in US Patent No 6,231,563 entitled "Directional Catheter". However, while the directional catheter of that patent addresses many problems associated with implantation in branching vessels by its application for manipulation of guidewires, the present invention allows movement of the side branch of a branched or bifurcated graft structure.

Figures 9a and 9b depict an intraluminal device 100 having a main body 101 and a side branch 102. Extending within the device 100 is a first tubular catheter 103 and a second tubular catheter 104.

Both tubular catheters 103, 104 are often incorporated within the one directional catheter device assembly, which is preloaded into the device 100. Each tubular body has an internal guidewire channel 119.

A primary guidewire 105a is within first tubular catheter 103, and secondary guidewire 105b is within the second tubular catheter 104. The second tubular catheter may be used to manoeuvre side branch 102 into a branching vessel.

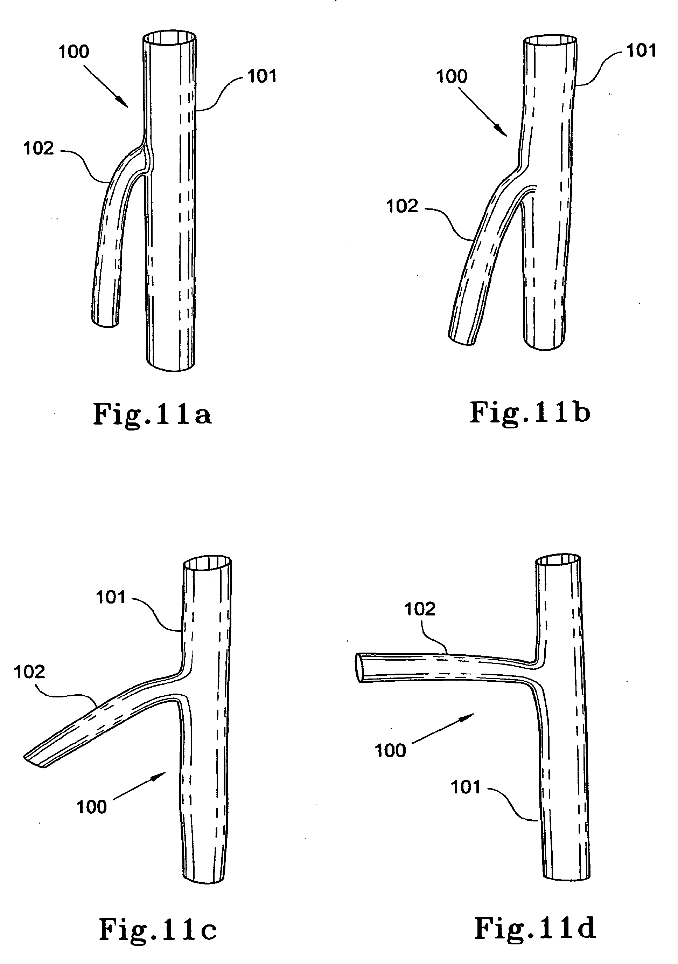

Side branch 102 is controllable through angles of 170° to 10° relative to the longitudinal axis Y of the main body 101. In Figure 9a, the depicted angle is approximately 80 degrees, and rotated to the right side. Side branch 102 may be moved to a number of orientations, with some examples depicted in Figures 11a to 12d.

One mechanism to manipulate side branch 102 is to manipulate secondary tubular catheter 104. Catheters 103 and 104 are also rotatable, to allow manipulation at all angles tangential to a vessel lumen.

Catheter 104 may be manipulated by a number of means. As depicted, guidewire 105b extends within and beyond the distal end 106 of catheter 104. Guidewire 105b may act as the steering mechanism to move the catheter 104 into a desired orientation. In this embodiment, while being sufficiently flexible to bend, the guidewire must also have sufficient strength to influence the orientation of both catheter 104 and side branch 102.

Primary guidewire 105a within the first tubular catheter 103 is relatively stiff and strong wire which directs passage of the graft and deployment sheath along an access vessel and into the desired position, such as within the thoracic or abdominal aorta. Wires of this type include an Amplatz 035 wire or Lunderquist 035 wire (both made and sold by Cook Inc.). Guidewire 105b within tubular catheter 104 is a relatively more flexible, non-kinkable wire which can be angulated and manipulated without causing it to kink or catch within the catheter.

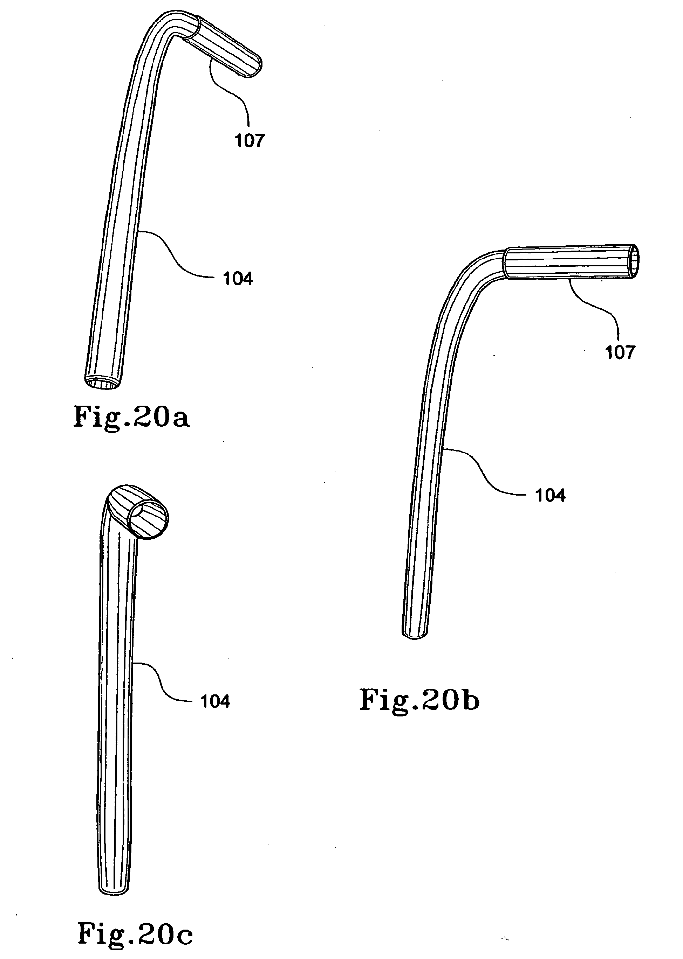

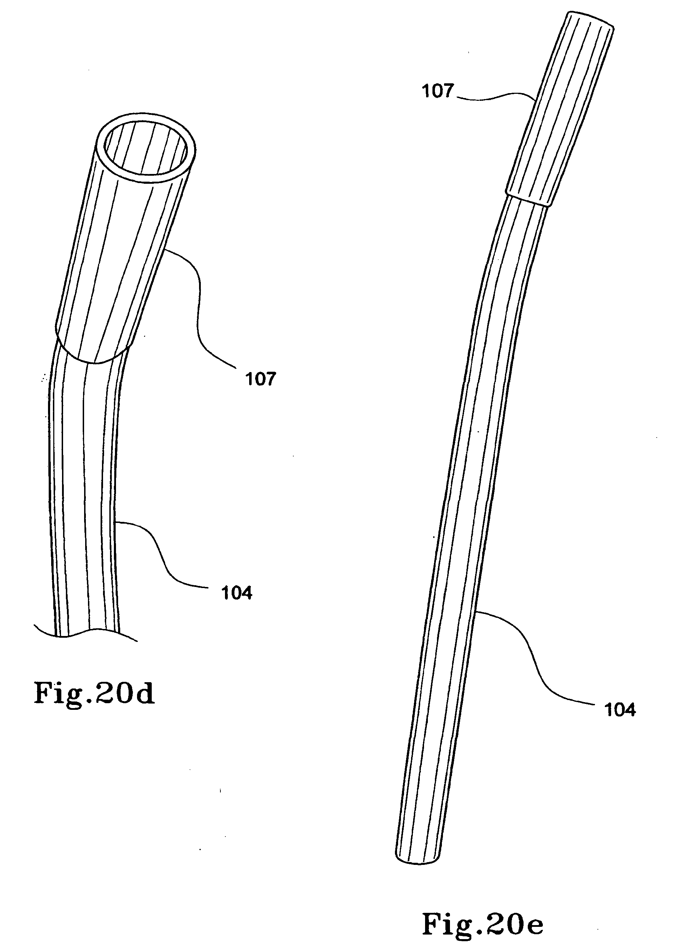

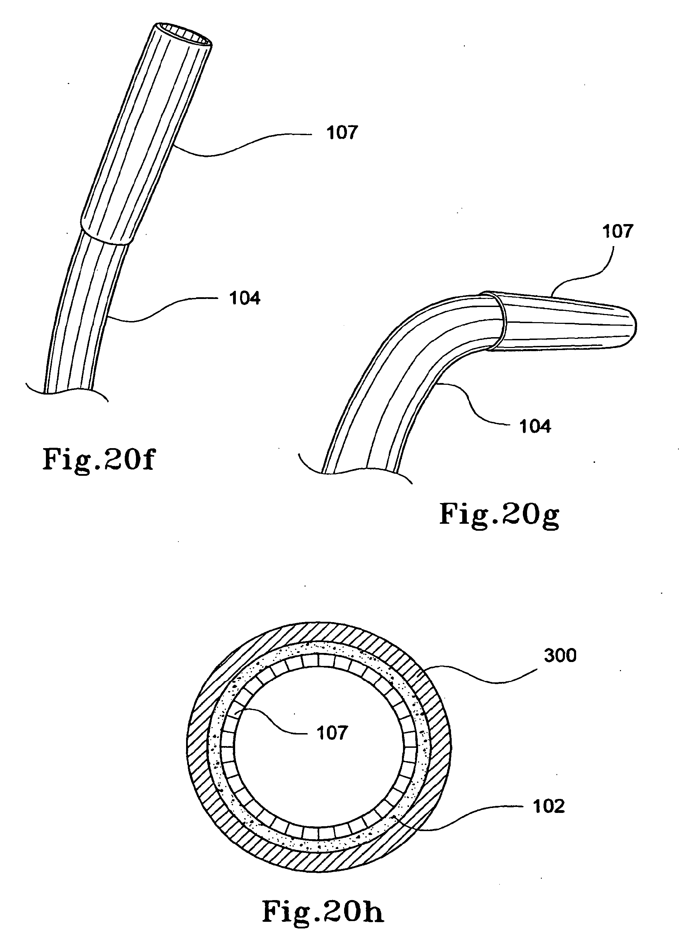

Rather than guidewire 105b steering catheter 104 and side branch 102, catheter 104 itself may comprise the steering mechanism. This embodiment is described in greater detail below in relation to Figures 20a to 20h.

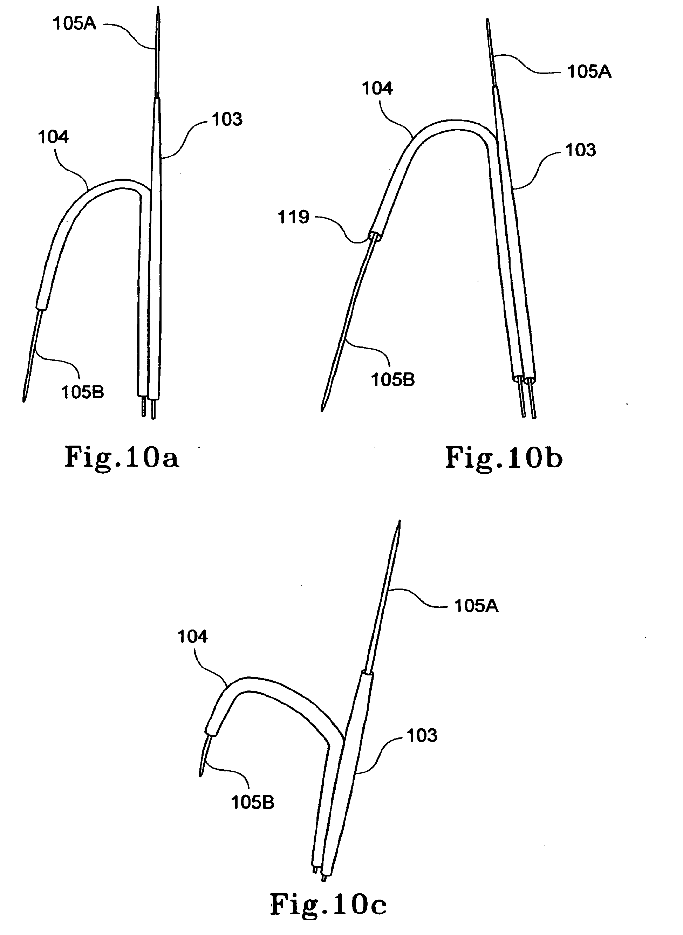

Figures 10a, b and c depict three perspectives of the two tubular catheters 103, 104 and their associated guidewires therein. The movement of the second tubular catheter 104 relative to the first tubular catheter 103 is one mechanism by which to move side branch 102.

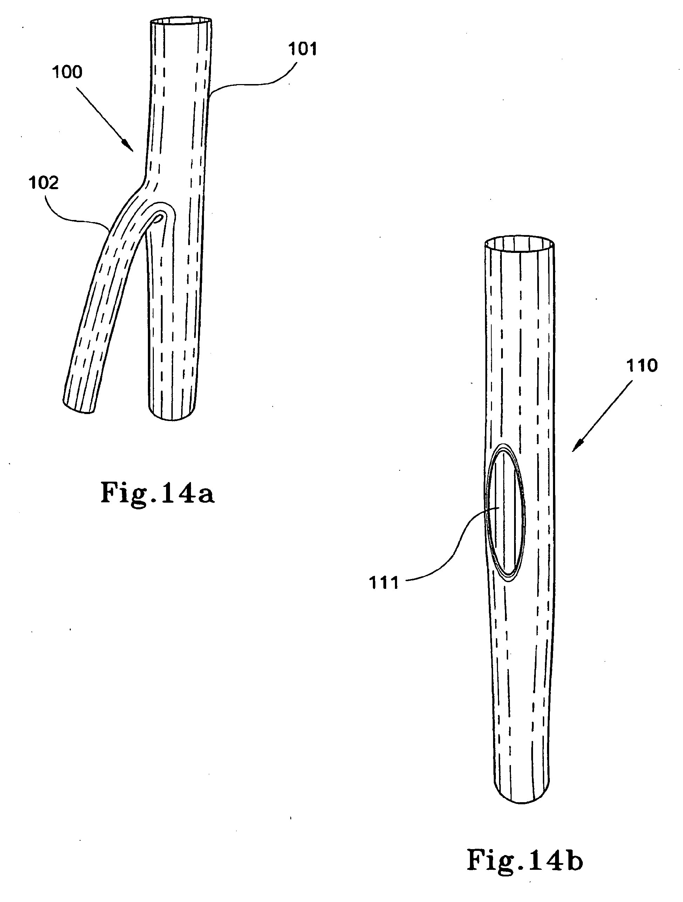

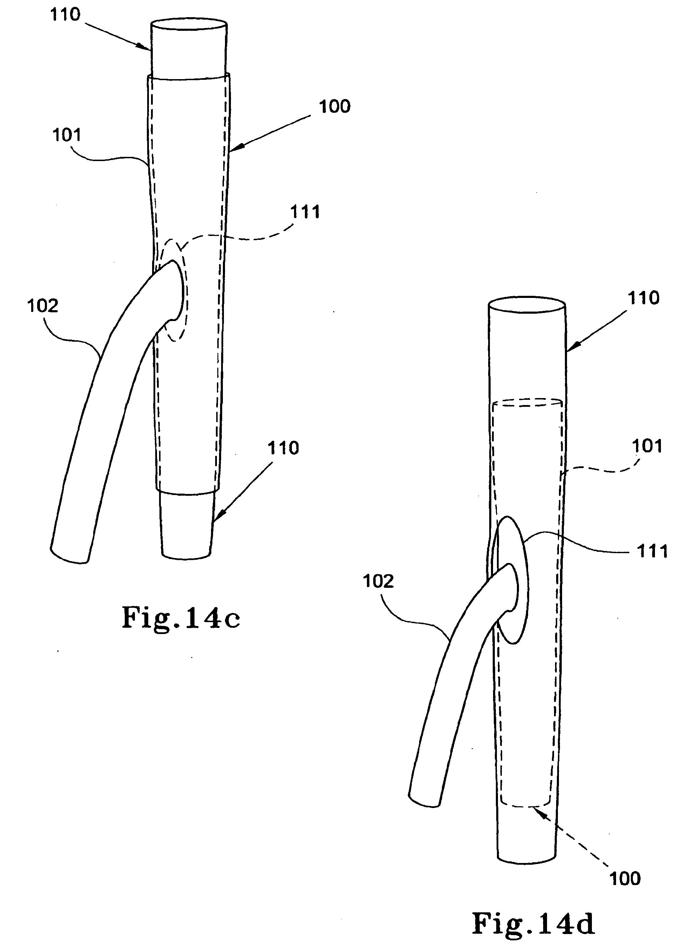

Figures 14b, 14c and 14d, depict a reinforcing secondary graft device 110 which extends through the lumen of the primary device 100 in Figure 14c. Secondary graft 110 has a large aperture 111 which is positioned over the opening to side branch 102 so that blood flow via side branch 102 will not be obstructed.

Side branch 102 as described above is preferably flexible in nature and typically capable of being compressed significantly. Side branch 102 therefore may not have much support. The double graft system depicted in Figure 14b to 14d to support the main body 101 of device 100. In Figure 14d the secondary graft is positioned first and the device 100 inserted therein.

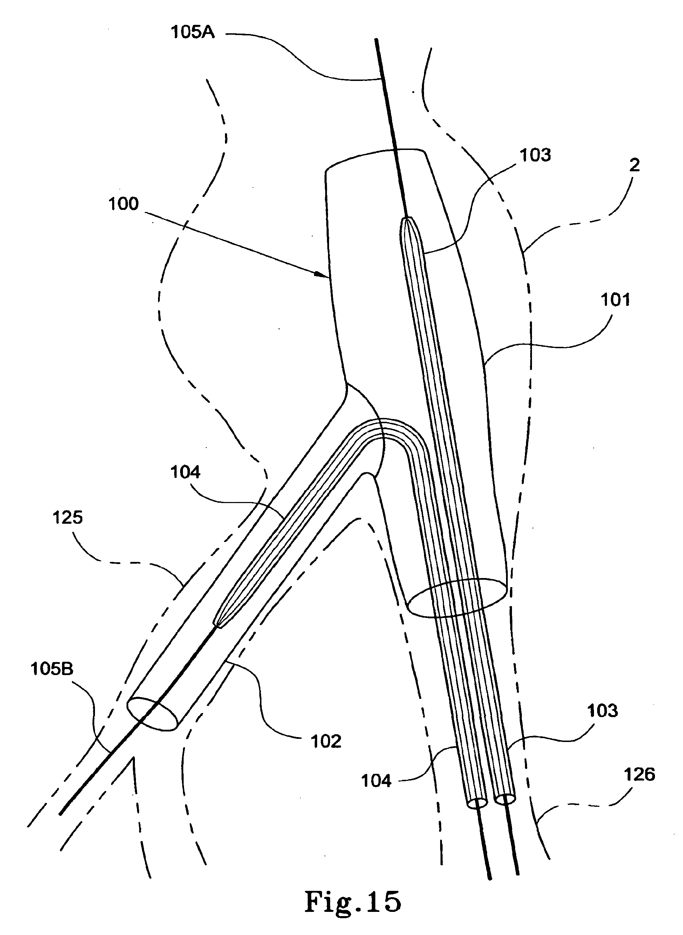

Figure 15 depicts side branch 102 in the internal iliac branch 125 and the main body 101 of the graft component in the left common iliac artery 2. This figure also depicts the method of access by a sheath in the ipsilateral femoral artery which is an extension of the external iliac artery (external sheath itself not shown).

A similar method and device as depicted here can be used to deploy branched grafts into major aortic branches such as the carotids, brachiocephalic, subclavians, renals or mesenteric arteries. For each of these applications, there may be variations in the diameter and length of the pre-branching components and the side-branch components. There may also be multiple side-branch components arising from the one pre-branching component (e.g. two branches, for steerable side-branch deployment into the carotid artery and the subclavian artery from the thoracic aorta arch).

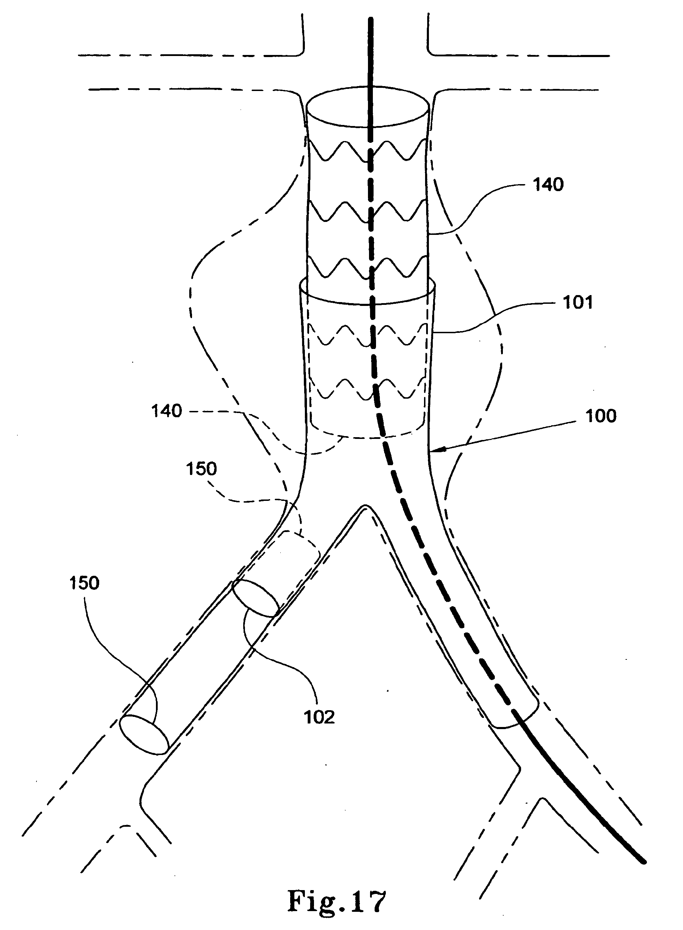

Figures 16a, 16b and 17 depict another embodiment of the invention in application for treatment of an abdominal aortic aneurysm (AAA). In this case, branch 102 is positioned and deployed into the right common iliac artery 3, while the main body 101 is bridging the aorta to the left common iliac artery 2, where the deployment sheath 120 is shown. This figure shows how the branched graft device can be positioned right on the bifurcation of the aorta 131, in contrast to known modular devices which sit higher within the AAA sac.

In Figure 17 an extension graft 150 is introduced to connect with side branch 102. An upstream graft 140 connects with main body 101.

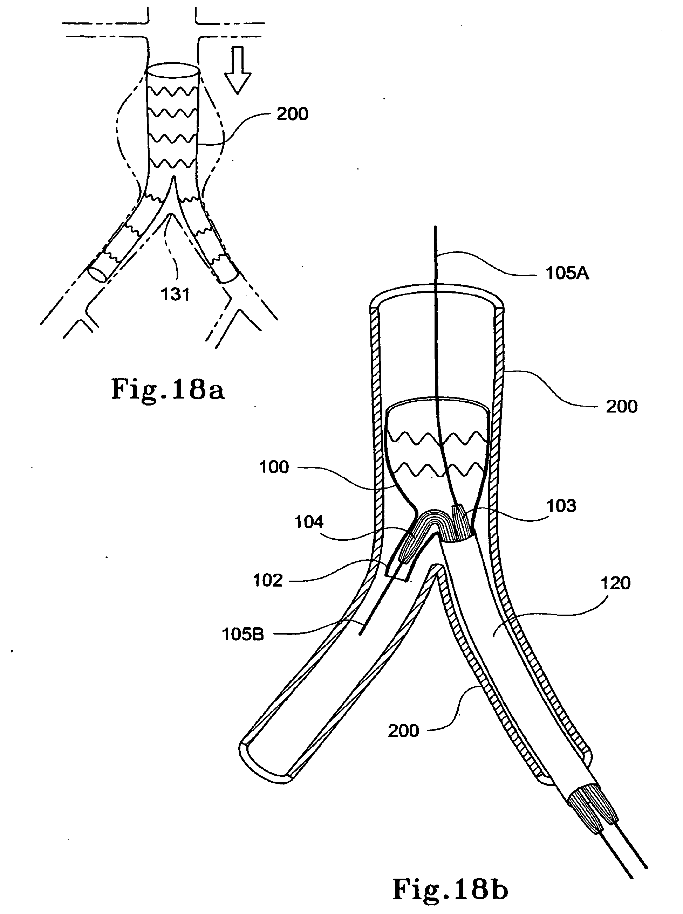

Figures 18a to 19b depict the re-lining of a prior-implanted endovascular graft 200 which has failed. Figure 18a depicts the graft sitting high above the bifurcation 131 of the aorta. In this application, branch 102 is initially manipulated into the contralateral limb of the prior bifurcated graft and the septum or crotch of the replacement device 100 positioned in close proximity to the septum of the prior graft 200, permitting good conformation of the new graft inside the prior failed graft.

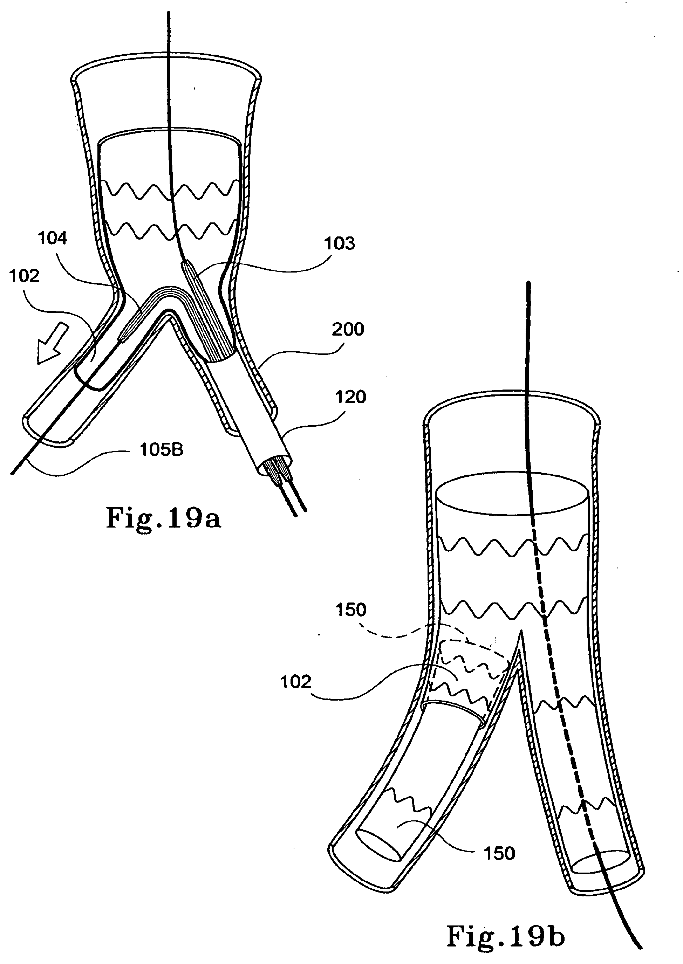

Figure 19b depicts the introduction of an extension graft 150 to engage with branch 102 to complete a salvage procedure.

Figures 20a) to g) show various perspectives of tubular catheter 104, with a manipulable tip 107. Manipulable tip is releasably connected to the remainder of catheter 104 such that following deployment, tip 107 is left within a post branching vessel 300 to form an inner sheath of branch portion 102. Figure 20h is a cross-sectional depiction of the tip 107 left *in situ.*

## Claims

1. An intraluminal device (10) comprising a tubular main body (12) extending from a first end to a second end, said main body (12) having a plurality of expandable wireforms;
wherein the wireforms are grouped in a first group of one or more self expandable wireforms (16) and a second group of one or more pressure expandable wireforms (17);
wherein the main body (12) comprises alternating groups of wireforms comprising said first group and said second group;
the device being configured such that, in use, a section of the tubular main body (12) is expanded outwards into conformability with a vessel wall to form at least one protrusion portion (20), the at least one protrusion portion (20) having a greater diameter than the remainder of the tubular main body (12), and the protrusion portion (20) being supported by one or more balloon expandable wireforms (17).

2. The intraluminal device of claim 1 wherein both the first group and the second group of wireforms (16,17) each comprise a single wireform.

3. The intraluminal device of claim 1 wherein the first group and the second group of wireforms (16,17) each comprise a plurality of wireforms.

4. The intraluminal device of any one of claims 1 to 3 wherein the wireforms (16,17) extend circumferentially around the tubular main body (12).

5. The intraluminal device of claim 1 wherein the wireforms (16,17) are radially compressible and expandable such that the tubular main body (12) is moveable between an insertion diameter, in which state the device (10) may be inserted intraluminally into a vessel and a larger, expanded diameter in which state the device (10) may be secured within the vessel.

## Patentansprüche

1. Intraluminale Vorrichtung (10), die einen rohrförmigen Hauptkörper (12) aufweist, der sich von einem ersten Ende zu einem zweiten Ende erstreckt, wobei der Hauptkörper (12) eine Vielzahl von expandierbaren Drahtformen aufweist;
wobei die Drahtformen zu einer ersten Gruppe von einer oder mehreren selbstexpandierbaren Drahtformen (16) und einer zweiten Gruppe von einer oder mehreren druckexpandierbaren Drahtformen (17) zusammengestellt werden;
wobei der Hauptkörper (12) abwechselnde Gruppen von Drahtformen aufweist, die die erste Gruppe und die zweite Gruppe aufweisen;
wobei die Vorrichtung so ausgebildet ist, dass bei Verwendung ein Abschnitt des rohrförmigen Hauptkörpers (12) nach außen in Übereinstimmung mit einer Gefäßwand expandiert wird, um mindestens einen Vorsprungsabschnitt (20) zu bilden, wobei der mindestens eine Vorsprungsabschnitt (20) einen größeren Durchmesser als der restliche rohrförmige Hauptkörper (12) aufweist, und wobei der Vorsprungsabschnitt (20) durch eine oder mehrere ballonexpandierbare Drahtformen (17) gestützt wird.

2. Intraluminale Vorrichtung nach Anspruch 1, bei der sowohl die erste Gruppe als auch die zweite Gruppe der Drahtformen (16, 17) jeweils eine einzelne Drahtform aufweist.

3. Intraluminale Vorrichtung nach Anspruch 1, bei der die erste Gruppe und die zweite Gruppe der Drahtformen (16, 17) jeweils eine Vielzahl von Drahtformen aufweist.

4. Intraluminale Vorrichtung nach einem der Ansprüche 1 bis 3, bei der sich die Drahtformen (16, 17) umfangsmäßig um den rohrförmigen Hauptkörper (12) erstrecken.

5. Intraluminale Vorrichtung nach Anspruch 1, bei der die Drahtformen (16, 17) radial zusammendrückbar und expandierbar sind, so dass der rohrförmige Hauptkörper (12) zwischen einem Durchmesser beim Einsetzen, wobei die Vorrichtung (10) in diesem Zustand intraluminal in ein Gefäß eingesetzt werden kann, und einem größeren expandierten Durchmesser beweglich ist, wobei die Vorrichtung (10) in diesem Zustand innerhalb des Gefäßes gesichert werden kann.

## Revendications

1. Dispositif intraluminal (10), comprenant un corps principal tubulaire (12) s'étendant d'une première extrémité vers une deuxième extrémité, ledit corps principal (12) comportant plusieurs formes de fil extensibles ;
dans lequel les formes de fil sont regroupées dans un premier groupe d'une ou plusieurs formes de fil auto-extensibles (16), et un deuxième groupe d'une ou plusieurs formes de fil extensibles par pression (17) ;
dans lequel le corps principal (12) comprend des groupes alternés de formes de fil comprenant ledit premier groupe et ledit deuxième groupe ;
le dispositif étant configuré de sorte qu'en service, une section du corps principal tubulaire (12) est étendue vers l'extérieur, en conformité avec une paroi de vaisseau, pour former au moins une partie en saillie (20), la au moins une partie en saillie (20) ayant un diamètre plus grand que la partie restante du corps principal tubulaire (12), et le partie en saillie (20) étant supportée par une ou plusieurs formes de fil extensibles par ballonnet (17).

2. Dispositif intraluminal selon la revendication 1, dans lequel le premier groupe et le deuxième groupe de formes de fil (16, 17) comprennent chacun une seule forme de fil.

3. Dispositif intraluminal selon la revendication 1, dans lequel le premier groupe et le deuxième groupe de formes de fil (16, 17) comprennent chacun plusieurs formes de fil.

4. Dispositif intraluminal selon l'une quelconque des revendications 1 à 3, dans lequel les formes de fil (16, 17) s'étendent de manière circonférentielle autour du corps principal tubulaire (12).

5. Dispositif intraluminal selon la revendication 1, dans lequel les formes de fil (16, 17) peuvent être comprimées et étendues radialement, de sorte que le corps principal tubulaire (12) peut être déplacé entre un diamètre d'insertion, le dispositif (10) pouvant dans cet état être inséré de manière intraluminale dans un vaisseau, et un diamètre étendu plus grand, le dispositif (10) pouvant dans cet état être fixé dans le vaisseau.
